# EUROPÄISCHE PATENTSCHRIFT

(11) **EP 2 623 070 B1**
(45) Veröffentlichungstag und Bekanntmachung des Hinweises auf die Patenterteilung: **30.04.2014**
(21) Anmeldenummer: 13002301.3
(22) Anmeldetag: 05.10.2010
(51) Int. Cl.: A61F 2/54, A61F 2/60

(54) **Verfahren zur Herstellung einer Kunstgliedhülle**
Method for manufacturing a covering for an artificial organ
Procédé de fabrication d'un enveloppe pour membre artificiel

(30) Priorität: 30.10.2009 DE 102009051441
(43) Veröffentlichungstag der Anmeldung: 07.08.2013
(62) Teilanmeldung aus: 10765961.7
(73) Patentinhaber: Otto Bock HealthCare GmbH, 37115 Duderstadt (DE)
(72) Erfinder: Janssen, Heinz-Gerd, 37115 Duderstadt (DE); Schneegans, Marcus, 37434 Rollshausen (DE); Schneegans, Waldemar, 37115 Duderstadt (DE)
(74) Vertreter: Stornebel, Kai

(56) Entgegenhaltungen:
- WO-A1-01/67842
- WO-A1-2009/115835
- GB-A- 2 341 325
- GB-A- 2 357 725

## Beschreibung

Die Erfindung betrifft ein Verfahren zum Herstellen einer Kunstgliedhülle.

Prothesen werden eingesetzt, um fehlende Gliedmaßen eines Körpers zu ersetzen. Hauptaufgabe der Prothese ist dabei in der Regel die beeinträchtigte oder verloren gegangene Funktion wiederherzustellen. Neben der rein funktionalen Komponente werden durch die Ersetzung der verloren gegangenen Gliedmaße auch optische bzw. kosmetische Funktionen erfüllt. Dazu ist vorgesehen, dass der funktionale Kern der Prothese von einer Umhüllung umgeben wird, die eine möglichst natürliche Anmutung bereitstellt, so dass es auf den ersten Blick nicht auffällt, dass eine Prothese getragen wird.

Fußprothesen wurden anfänglich aus einem Holzkern herausgearbeitet. Ein solcher Holzkern wurde dann zur Verbesserung der Widerstandsfähigkeit gegen Wasser mit einem Wasser abweisenden, elastischen Material umklebt. Dies ist beispielsweise in der US 4,180,870 beschrieben. Die technische Ausgestaltung des Holzkernes beschränkte sich auf eine reine Abstütz- und Abrollfunktion.

In der US 3,953,990 ist eine Beinprothese mit einem Schienbeinbereich, einem Oberschenkelbereich und einem Kniegelenksbereich gezeigt. Alle Bereiche sind mit separaten Umhüllungen umgeben, wobei die aneinander angrenzenden Umhüllungen einander überlappen. Der Prothesenfuß sieht keine Umhüllung vor.

Die US 4,007,496 beschreibt eine Verbindung zwischen einer Unterschenkelumhüllung und einem Prothesenfuß. Auch hier ist nicht vorgesehen, dass der Prothesenfuß eine kosmetische Umhüllung aufweist.

Die GB 2 357 725 beschreibt eine kosmetische Umhüllung aus Silikon für eine Beinprothese. Die kosmetische Umhüllung weist einen hohlen Fußbereich und einen hohlen Schienbeinbereich auf. Das Material der Umhüllung kann auf die Prothese aufgerollt werden, so dass eine Konfektionsgröße der Umhüllung für die üblicherweise benutzten Prothesen ausreichend ist. Im Schienbeinbereich kann ein Silikonmaterial mit einer Härte eingesetzt werden, die größer als die Härte des Materials im Wadenbereich ist. Die Kunstgliedhülle wird an den Schienbeinbereich angeformt. Die Kunstgliedhülle kann dabei eine Härte aufweisen, die von der Härte des Materials für den Unterschenkelbereich abweicht.

Die GB 2 341 325 beschreibt ein Verfahren zum Herstellen einer Prothesen- oder Orthesenumhüllung. Dabei werden in mehreren Schichten unterschiedliche Färbungen aufgebracht, um eine Gesamterscheinung zu erhalten, die der natürlichen Haut ähnlich ist. Der Fußteil der Umhüllung ist an das Unterschenkelteil angeformt.

Die WO 2005/117746 A2 beschreibt eine funktionale Kunstgliedhülle mit einer Öffnung am oberen Ende und einem Hohlraum zur Aufnahme eines Prothesenfußes. Die Wand, die den Hohlraum umgibt, weist die Gestalt eines natürlichen menschlichen Fußes auf und bildet unter anderem einen Bodenteil mit einem Sohlenbereich aus, der einen Zehenabschnitt, einen Fersenabschnitt und einen Mittelfußabschnitt aufweist. In dem Sohlenbereich sind unterschiedliche Steifigkeitsgrade ausgebildet, um ein gewünschtes Abrollverhalten von der Ferse zum Zeh sowie von der lateralen zur medialen Seite zu erreichen. Dazu ist beispielsweise vorgesehen, dass Einsätze in dem Fersen- und Ballenbereich eingearbeitet sind. Ebenfalls können steife Elemente an der Umhüllung befestigt werden.

Die WO 2009/115835 A1 betrifft eine Prothesenverkleidung mit einer Außenschicht, die die äußere Oberfläche der Prothesenverkleidung ausbildet und einer Innenschicht mit einer inneren Oberfläche, die an der Prothese anliegt. Die Außen- und Innenschichten haben im Wesentlichen die gleiche Form und liegen unmittelbar aneinander an. Die beiden Schichten sind miteinander an einer Vielzahl von einander beabstandeten Stellen verbunden und sind darüber hinausgehend nicht miteinander verbunden. Eine der Schichten kann als Elastomer und die andere als Teil eines Textil ausgebildet sein.

Die EP 0 281 855 A1 betrifft eine Innenhand als orthopädisches Passteil aus einem thermoplastischen Kunststoff zur Aufnahme einer Handmechanik mit einer am Handabschluss eingearbeiteten Ringnut, in die ein die Innenhand auf der Handmechanik fixierender Ring eingelegt ist. Zur Verbesserung der Funktion der Innenhand wird eine 2-Komponenten-Sandwich-Bauweise vorgeschlagen, wobei der Nutzungsbereich zumindest des Daumens und des Zeigefingers einen höheren Härtegrad aufweist als der übrige Handkörper, insbesondere einen höheren Härtegrad als in dem Gelenkbereich zwischen dem Daumen und Zeigefinger.

Aufgabe der vorliegenden Erfindung ist es, Verfahren zur Herstellung einer Kunstgliedhülle bereitzustellen, die eine hohe Verschleißfestigkeit aufweist, ausreichend elastisch ist, um z.B. bei Prothesenfüßen mit einstellbarer Absatzhöhe eine ausreichende Anpassbarkeit zu ermöglichen und darüber hinaus kompakt aufgebaut ist, um möglichst wenig aufzutragen.

Erfindungsgemäß wird diese Aufgabe durch ein Verfahren mit den Merkmalen des Hauptanspruches. Vorteilhafte Ausgestaltungen und Weiterbildungen der Erfindung sind in den Unteransprüchen aufgeführt.

Das erfindungsgemäße Verfahren zur Herstellung einer Kunstgliedhülle sieht vor, dass auf die Außenseite eines hohlen Elastomerträgers, der die Form eines natürlichen Körpergliedes aufweist, eine textile Zwischenschicht aufgebracht wird, auf die eine Silikonschicht aufgebracht, insbesondere aufgetragen wird. Diese textile Zwischenschicht verbessert die Haftfähigkeit des Silikonüberzuges an dem Trägermaterial. Darüber hinaus kann die textile Zwischenschicht die Weiterreißfähigkeit verringern, so dass insgesamt eine höhere Festigkeit und Haltbarkeit der Kunstgliedhülle erreicht wird. Als Zwischenschicht kann insbesondere eine Flausch-, Beflockung- oder Veloursschicht aufgebracht werden. Die Ausgestaltung als eine Flausch- oder Veloursschicht hat den Vorteil, dass keine gewebte oder gewirkte Struktur in der Zwischenschicht vorhanden ist, so dass die Elastizität des Werkstoffes nicht eingeschränkt wird; insbesondere im Bereich der Einführöffnung ist dies von Vorteil.

Die textile Zwischenschicht ist formschlüssig mit der Silikonschicht und dem Elastomerträger verbunden, so dass auch bei Verwendung von Materialien, die nicht aneinander haften, eine sichere Verbindung zwischen dem Trägermaterial und der Silikonschicht erreicht wird.

Der Elastomerträger kann aus einem Polyurethan ausgebildet sein, auf dessen Außenseite die Textilschicht aufgebracht wird.

Die erfindungsgemäße Kunstgliedhülle für ein Kunstglied mit einer Einführöffnung zum Einführen des Kunstgliedes, wobei an der Kunstgliedhülle ein Knöchelbereich ausgebildet ist, an dem sich ein Endbereich einstückig anschließt, sieht vor, dass die Kunstgliedhülle aus einem Elastomerwerkstoff, z.B. einem Polyurethanwerkstoff ausgebildet ist, der im Gelenkbereich, bei einer Prothesenfußhülle insbesondere im Knöchelbereich, eine geringere Shore-Härte als im Endbereich, bei der Prothesenfußhüllen im Sohlenbereich, aufweist. Bei Prothesenfüßen mit einstellbarer Absatzhöhe ist es notwendig, dass der Knöchelbereich der Kunstgliedhülle ausreichend beweglich ist, um die unterschiedlichen Grundeinstellungen des Prothesenfußes mitmachen zu können. Bei Prothesenhänden ist eine hohe Beweglichkeit und Elastizität aufgrund des große Bewegungsumfanges und der vielfältigen Bewegungsmöglichkeiten der Prothesenhand notwendig. Üblicherweise werden daher für Kunstgliedhüllen Schaumwerkstoffe eingesetzt, die jedoch keine Abdichtung des Kunstgliedes im Gelenkbereich gegen das Eindingen von Wasser oder Schmutz ermöglichen. Darüber hinaus trägt der Schaumwerkstoff auf und weist nur eine geringe Verschleißfestigkeit auf. Eine Ausgestaltung der Kunstgliedhülle aus Silikonwerkstoff ist nachteilig, da der Weiterreißwiderstand bei Silikon sehr gering ist. Bildet sich ein Riss innerhalb der Silikonumhüllung aus, vergrößert sich dieser sehr leicht. Erfindungsgemäß ist daher ein Elastomerwerkstoff, z.B. eine Polyurethanwerkstoff vorgesehen, der eine wesentlich bessere Weiterreißfestigkeit als die üblicherweise verwendeten Silikonwerkstoffe aufweist. Problematisch dabei ist jedoch, dass die Elastomerwerkstoffe, insbesondere Polyurethanwerkstoffe nur eine geringe Elastizität aufweisen, insbesondere wenn sie so ausgelegt sind, dass sie eine ausreichende Verschleißfestigkeit für die hoch beanspruchten Bereiche in der Ferse, am Ballen oder an den Knöcheln oder Fingerspitzen aufweisen. Erfindungsgemäß ist daher vorgesehen, dass im Endbereich, also z.B. im Handflächen- und Fingerbereich sowie im Sohlenbereich ein wesentlich härterer Elastomerwerkstoff eingesetzt wird als im Gelenkbereich, so dass die Kunstgliedhülle oder Kunstgliedkosmetik im Endbereich fest und stabil ist, während im Bereich der Einführöffnung eine weiche und elastische Ausgestaltung des Elastomerwerkstoffes vorhanden ist.

Vorteilhafterweise weist der Elastomerwerkstoff, z.B. Polyurethanwerkstoff im Gelenkbereich eine Härte von 15 bis 20 Shore A auf, während im Endbereich, z.B. im Sohlenbereich der Elastomerwerkstoff eine Härte von 45 bis 75 Shore A aufweist. Auf diese Weise ist es möglich, einerseits die Festigkeits- und Haltbarkeitsanforderungen der Kunstgliedhülle zu erfüllen und andererseits die gewünschte Elastizität im Bereich der Einführöffnung und im Gelenk- oder Knöchelbereich bereitzustellen.

Eine weitere Verbesserung der Elastizität wird erreicht, wenn im Gelenk- oder Knöchelbereich eine Faltenbalgstruktur ausgebildet ist. Bei einem Prothesenfuß erstreckt sich der Knöchelbereich sowohl in proximaler als auch distaler Richtung der Schwenkachse des Prothesenfußes, die in der Regel im Bereich des Köchels eines natürlichen Fußes liegt. Der Knöchelbereich der Prothesenfußhülle erstreckt sich folglich auch in proximaler Richtung, also oberhalb der Schwenkachse des Prothesenfußes. Eine entsprechende Ausgestaltung ist bei anderen Kunstgliedern vorzusehen, beispielsweise bei Prothesenhänden, bei denen der Gelenkbereich im Bereich des natürlichen Handgelenkes ausgebildet ist und sich sowohl in Richtung auf die Mittelhand als auch in Richtung auf den Unterarm erstreckt. Die Faltenbalgstruktur ermöglicht es z.B., dass die Absatzhöhe im Wesentlichen frei eingestellt und die Prothesenhand frei bewegt werden können. Ebenfalls ist es möglich, durch die Faltenbalgstruktur in der Kunstgliedhülle eine Materialstreckung bzw. -stauchung während der Bewegung zu vermindern, so dass neben einer erleichterten Einstellbarkeit auch eine erhöhte Dauerfestigkeit der Kunstgliedhülle erzielt wird.

Bevorzugt ist vorgesehen, dass im Endbereich, insbesondere im Sohlenbereich, der Elastomerwerkstoff eine gleichmäßige Härte aufweist, um ein gleichmäßiges Materialgefüge zu erhalten. Dadurch wird vermieden, dass Trennfugen oder Sollbruchstellen durch Materialübergänge ausgebildet werden.

Eine weitere Verbesserung der Elastizität wird dadurch erreicht, dass im Gelenkbereich eine geringere Materialstärke als im Endbereich vorhanden ist, wobei in besonders belasteten Bereichen des Endbereiches auch Materialverstärkungen vorgesehen sein können. Solche Materialverstärkungen sind insbesondere im Fersenbereich, Ballenbereich, Zehenbereich, Fingerspitzenbereich und/oder Handflächenbereich vorhanden. Gegebenenfalls kann in allen diesen Bereichen eine Materialverstärkung vorgesehen sein. Sollte sich aufgrund der Beschaffenheit des Kunstgliedes oder angepasster Komponenten herausstellen, dass an einigen Stellen nur eine geringe Materialbelastung zu erwarten ist, kann die Materialverstärkung auch nur in Teilen dieser Bereiche vorhanden sein.

Die Härte des Elastomerwerkstoffes kann von dem Endbereich zu dem Gelenkbereich kontinuierlich abnehmen. Alternativ dazu ist vorgesehen, dass verschiedene Zonen abnehmender Werkstoffhärte vom Endbereich zum Gelenkbereich vorgesehen sind. Die Kunstgliedhülle weist somit Zonierungen auf, die unterschiedliche Materialstärken, Materialhärten oder auch Färbungen aufweisen, so dass eine Anpassung der Kunstgliedhülle an die jeweiligen Einsatzzwecke erfolgen kann.

Eine Weiterbildung der Erfindung sieht vor, dass an der Einführöffnung der Kunstgliedhülle ein Dichtungsring, insbesondere ein so genannter O-Ring eingebettet ist, um die Dichtung zwischen der Kunstgliedhülle und dem Kunstglied oder zwischen der Kunstgliedhülle und dem Schaftbereich eines oberen Anschlussteils zu verbessern. Dadurch wird die Störanfälligkeit der Kunstglieder verringert, da ein Wassereintritt wirksam verhindert werden kann.

An der Außenseite des Elastomerwerkstoffes, insbesondere Polyurethanwerkstoffes kann ein Silikonüberzug aufgebracht oder aufgetragen sein, um eine verbesserte Optik und eine verbesserte Haptik zu erreichen, da der Silikonüberzug eine sehr natürliche Oberfläche aufweist.

Eine weitere Verbesserung der Erfindung sieht vor, dass zwischen dem Silikonüberzug und dem Elastomerwerkstoff eine textile Zwischenschicht angeordnet ist, insbesondere eine Flauschschicht, eine Veloursschicht oder eine Beflockungsschicht, um die Haftung des Silikons an dem Elastomerwerkstoff zu verbessern. Neben der verbesserten Haptik durch den Silikonüberzug würde auch eine verbesserte Optik bereitgestellt, da der Silikonüberzug besser an das natürliche Erscheinungsbild angepasst werden kann. Die textile Zwischenschicht verbessert die Haftfähigkeit des Silikons an den Elastomerwerkstoff, insbesondere wenn die textile Zwischenschicht in dem Elastomerwerkstoff eingebettet ist und der Silikonwerkstoff sich mit dem nicht eingebetteten Teil der textilen Zwischenschicht formschlüssig verbinden kann. Die Flauschschicht oder textile Zwischenschicht kann darüber hinaus genutzt werden, um die natürliche Anmutung des Kunstgliedes zu verbessern, indem die Farbgebung der Zwischenschicht an die Gestaltung einer natürlichen Hautoberfläche angepasst wird.

Der Silikonüberzug kann auch auf der beschichteten Kunstgliedhülle aufgeklebt werden. Der Silikonüberzug kann separat hergestellt und über eine Verklebung mit der textilen Zwischenschicht, zum Beispiels einer Flauschschicht verbunden werden. Die textilbeschichtete Kunstgliedhülle wird hierzu mit einem Silikonkleber bestrichen, besprüht oder anderweitig benetzt, z.B. getaucht, anschließend wird die Silikonkosmetik oder der Silikonüberzug übergerollt oder übergezogen. Alternativ kann die Silikonschicht bzw. der Silikonüberzug in einem Silikonbad aufgebracht werden. Als Haftvermittler zwischen der Textilschicht und dem Silikonüberzug oder dem Silikonkleber sind in der Regel flüssige Silikone vorgesehen, wobei die Aufbringung des Haftvermittlers in einem Tauchbad erfolgen kann. Die Haftvermittler gehen mit der Textilbeschichtung eine formschlüssige und mit dem Silikonüberzug eine stoffschlüssige Verbindung ein.

Die Anordnung der textilen Zwischenschicht zwischen dem Trägermaterial, insbesondere Polyurethan, und der Silikonschicht ist auch für sich genommen eine Verbesserung gegenüber dem Stand der Technik, auch ohne die Ausgestaltung der verschiedenen Bereiche der Kunstgliedhülle mit verschiedenen Härtegraden. Die obigen Ausführungen zu der Kunstgliedhülle lassen sich auch auf Ausgestaltungen mit einer textilen Zwischenschicht übertragen, bei denen auf unterschiedliche Härtegrade verzichtet wurde.

Nachfolgend wird ein Ausführungsbeispiel der Erfindung anhand der beigefügten Figuren näher erläutert. Es zeigen:
- Fig. 1: - eine Fußhülle in Seitenansicht;
- Fig. 2: - eine Fußhülle in einer perspektivischen Schrägdraufsicht;
- Fig. 3: - eine Fußhülle in Draufsicht;
- Fig. 4: - eine Variante der Erfindung mit einem Silikonüberzug; sowie
- Fig. 5: - eine Fußhülle an einer Unterschenkelprothese.

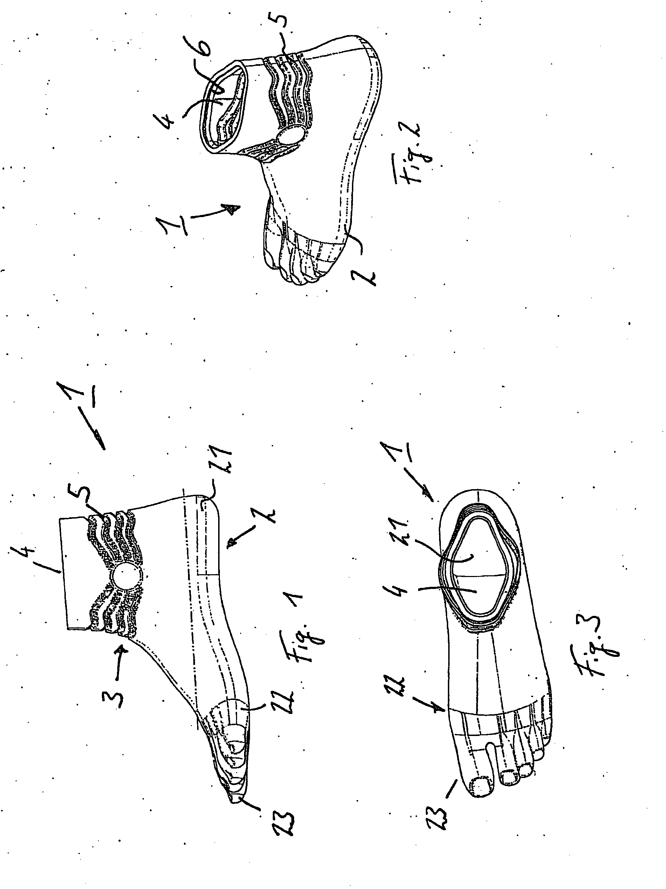

In der Figur 1 ist eine Kunstgliedhülle 1 in Gestalt einer Fußhülle mit einem als Sohlenbereich ausgestalteten Endbereich 2 und einem als Knöchelbereich ausgestalteten Gelenkbereich 3 dargestellt. Innerhalb der Fußhülle 1 ist ein nicht dargestellter Hohlraum ausgebildet, in dem ein ebenfalls nicht dargestellter Prothesenfuß eingeführt wird. Das Einführen erfolgt durch die am oberen Ende ausgebildete Einführöffnung 4. An der Einführöffnung 4 schließt sich der Knöchelbereich 3 an, der in der dargestellten Ausführungsform eine Faltenbalgstruktur 5 aufweist, die sich oberhalb und unterhalb einer Knöchelgelenkachse erstreckt. Um diese Achse, deren Position in der Fig. 1 als ein runder Bereich dargestellt ist, kann der Prothesenfuß verschwenkt werden, beispielsweise um die Absatzhöhe einzustellen, wenn der Prothesenträger ein Schuhmodell wechselt, das eine andere Absatzhöhe aufweist als das vorherige.

Der Knöchelbereich 3 weist eine Materialhärte von 15 bis 25 Shore A auf, während sich der einstückig daran anschließende Sohlenbereich 2 eine Materialhärte zwischen 45 und 75 Shore A aufweist. Der Übergang der jeweiligen Shore-Härte vom Knöchelbereich 3 zum Sohlenbereich 2 kann fließend oder diskret verlaufen, beispielsweise in mehreren Stufen, so dass sich im unteren Sohlenbereich die größte Härte ergibt, sich daran anschließt ein Bereich mit einer etwas geringeren Shore-Härte, wohingegen im Knöchelbereich 3 und insbesondere im Bereich der Einführöffnung 4 die niedrigste Shore-Härte einstellt.

Über den Bereich der Längserstreckung des Sohlenbereiches 2 können in unterschiedlichen Bereichen unterschiedliche Materialstärken ausgebildet sein. Im Fersenbereich 21 kann zur Verbesserung der Dauerhaltbarkeit eine größere Materialstärke an dem Elastomerwerkstoff, z.B. Polyurethan vorgesehen sein als im Mittelfußbereich, gleiches gilt für den Ballenbereich 22 oder den Zehenbereich 23, die ebenfalls zu den höher belasteten Bereichen der Fußhülle 1 zählen.

In der Figur 2 ist in der perspektivischen Darstellung deutlich der Faltenbalgbereich 5 um die Knöchelachse herum zu erkennen. Ebenfalls ist zu erkennen, dass in der Einführöffnung 4 im oberen Bereich ein Dichtungsring 6 eingebracht ist, der eine sichere Abdichtung gegenüber einem Feuchtigkeitseintritt gewährleistet. Der Dichtungsring 6 kann entweder eingeformt oder in eine Ausnehmung eingelegt sein. Da sich der Knöchelbereich 3 über die Knöchelachse hinaus in Richtung auf ein Unterschenkelteil erstreckt, liegt der Dichtungsring 6 an einem Unterschenkelteil an und verhindert ein Eindringen von Schmutz und Feuchtigkeit von oben.

In der Draufsicht gemäß Figur 3 sind die unterschiedlichen Sohlenbereiche, nämlich Fersenbereich 21, Ballenbereich 22 und Zehenbereich 23 zu erkennen. Ebenfalls ist zu erkennen, dass im dargestellten Ausführungsbeispiel im Fersenbereich 21 eine größere Materialstärke als im Mittelfußbereich ausgebildet ist. Die Formgestaltung der Fußhülle entspricht der eines natürlichen Fußes, abgesehen davon, dass nur die Großzehe einzeln ausgebildet ist, während die übrigen Zehen als eine zusammenhängende Zehenanordnung ausgebildet sind.

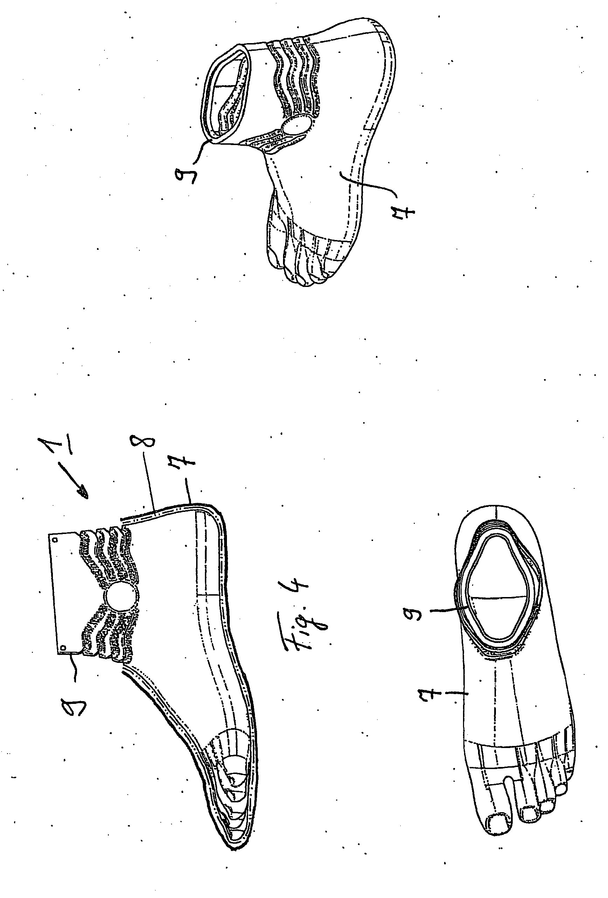

In der Figur 4 ist in verschiedenen Ansichten, z.B. oben links in einer schematischen Querschnittansicht eine Variante der Erfindung gezeigt, bei der auf den Polyurethanträger 9der Fußhülle, wie sie in den Figuren 1 bis 3 gezeigt ist, eine Flauschschicht 8 als textile Zwischenschicht aufgebracht ist. Diese textile Zwischenschicht 8 ist mit dem Polyurethanwerkstoff der Fußhülle 1 verbunden, entweder über eine Klebeverbindung oder über ein formschlüssiges Einbetten in dem Polyurethanwerkstoff. In dem dargestellten Ausführungsbeispiel erstreckt sich die textile Zwischenschicht 8 nur bis zum unteren Bereich des Faltenbalgbereiches 5. Auf der Außenseite der textilen Zwischenschicht 8 ist eine Silikonschicht 7 aufgebracht, die eine hautähnliche Textur aufweist und den äußeren Abschluss der Fußhülle 1 darstellt. Die Silikonaußenschicht 7 ist formschlüssig mit der textilen Zwischenschicht 8 verbunden, so dass auch Komponenten dauerhaft aneinander gebunden werden können, die ohne die textile Zwischenschicht 8 nicht aneinander haften würden. Die Silikonschicht 7 bildet den äußeren Abschluss der Fußhülle 1, da diese einer hautähnlichen Struktur und Haptik sehr nahe kommt. Nachteilig an einer Silikonschicht 7 an der Außenseite ist die Tatsache, dass sie nur einen geringen Weiterreißwiderstand aufweist. Der benötigte Weiterreißwiderstand wird durch den Polyurethanträger 9 bereitgestellt. Darüber hinaus wird durch die textile Zwischenschicht 8 die Weiterreißfähigkeit weiter verringert.

Die Silikonschicht 7 ist in dem dargestellten Ausführungsbeispiel auf dem Polyurethanträger 9 aufgeklebt. Dazu wird der Silikonüberzug separat hergestellt und über eine Verklebung mit der textilen Zwischenschicht 8, zum Beispiels einer Flauschschicht verbunden werden. Die textilbeschichtete Kunstgliedhülle wird hierzu mit einem Silikonkleber bestrichen, besprüht oder anderweitig benetzt, z.B. getaucht, anschließend wird die Silikonschicht 7 übergerollt oder übergezogen. Alternativ kann die Silikonschicht 7 in einem Silikonbad aufgebracht werden. Als Haftvermittler zwischen der textilen Zwischenschicht 8 und der Silikonschicht 7 oder dem Silikonkleber sind in der Regel flüssige Silikone vorgesehen, wobei die Aufbringung des Haftvermittlers in einem Tauchbad erfolgen kann. Die Haftvermittler gehen mit der textilen Zwischenschicht 8 eine formschlüssige und mit der Silikonschicht 7 eine stoffschlüssige Verbindung ein.

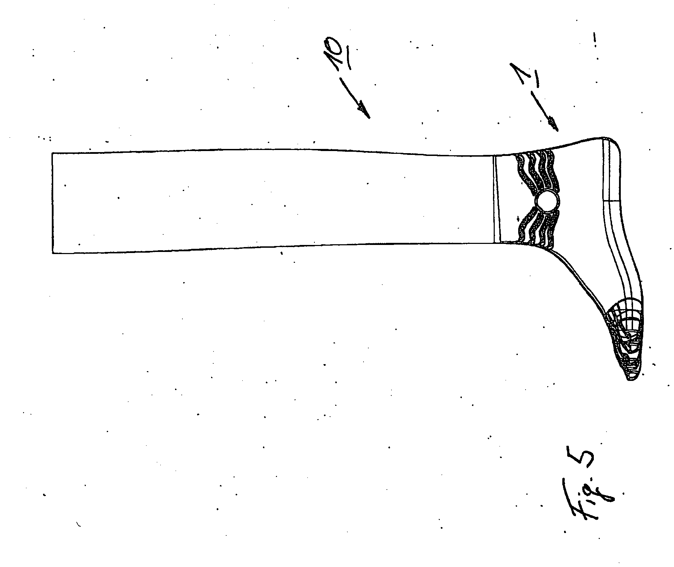

In der Figur 5 ist eine fertig montierte Fußhülle 1 an einem Unterschenkelschaft 10 gezeigt. Der Unterschenkelschaft 10 ist ebenfalls mit einer Kosmetik versehen, die in der optischen Anmutung der Fußhülle 1 entspricht. Bevorzugt ist die kosmetische Außenhülle des Unterschenkelschaftes 10 aus einem Silikonwerkstoff ausgebildet, andere Werkstoffe sind ebenfalls möglich. Es wird ein im Wesentlichen nahtloser Übergang zwischen der Fußhülle 1 und der Unterschenkelkosmetik 10 bereitgestellt. Wird die Absatzhöhe der Unterschenkelprothese verändert, kann über die Faltenbalgstruktur 5 leicht der Ausgleich erfolgen, ohne dass es zu Stauchungen oder hohen Zugbelastungen in der Fußkosmetik 1 kommt.

Die erfindungsgemäße Fußhülle 1 weist neben den sehr guten technischen Eigenschaften durch den Polyurethanträger 9 auch sehr gute optische Eigenschaften auf, da die Fußkosmetik mit dem Silikonüberzug 7 sicher an der Fußhülle 1 gehalten ist und dadurch keine Falten schlägt.

Sofern die textile Zwischenschicht 8 nicht als ein Gewebe, Gewirk oder Gestrick ausgebildet ist, wird die Elastizität der Fußhülle 1 nicht beeinträchtigt. Darüber hinaus kann durch die Flausch- oder Veloursbeschichtung bereits eine Farbgebung erfolgen, die als Untergrund für die Silikonschicht 7 dienen kann. Die Textilschicht 8 sorgt für einen gleichmäßigen Klebeauftrag.

Die Textilschicht 8 wird nach dem Herstellen oder beim Herstellen des Polyurethanträgers 9 aufgebracht, beispielsweise indem ein Flauschmaterial in die Negativform des Fußrohlinges eingelegt wird. Dann wird das Polyurethanmaterial in die Form eingefüllt und ausgehärtet. Nach dem Entnehmen des Rohlings kann dann die Silikonschicht 7 als Abschlussschicht aufgebracht werden, beispielsweise durch Tränken in einem Silikonbad oder dergleichen.

## Patentansprüche

1. Verfahren zur Herstellung einer Kunstgliedhülle (1), bei dem auf die Außenseite eines hohlen Elastomerträgers (9) eine textile Zwischenschicht (8) aufgebracht wird, **dadurch gekennzeichnet, dass** auf die textile Zwischenschicht (8) eine Silikonschicht (7) aufgebracht wird.

2. Verfahren nach Anspruch 1, **dadurch gekennzeichnet, dass** der Elastomerträger (9) als Polyurethanträger ausgebildet und auf die Außenseite des hohlen Polyurethanträgers die textile Zwischenschicht (8) aufgebracht wird, auf die die Silikonschicht (7) aufgebracht wird

3. Verfahren nach Anspruch 1 oder 2, **dadurch gekennzeichnet, dass** als Zwischenschicht (8) eine Flausch-, Beflockungs- oder Veloursschicht aufgebracht wird.

4. Verfahren nach einem der Ansprüche 1 bis 3, **dadurch gekennzeichnet, dass** die textile Zwischenschicht (8) formschlüssig mit der Silikonschicht (7) und dem Polyurethanträger (9) verbunden wird.

## Claims

1. A method for producing an artificial limb casing (1), in which a textile intermediate layer (8) is applied to the outer side of a hollow elastomer substrate (9), **characterized in that** a silicone layer (7) is applied to said textile intermediate layer (8).

2. The method as claimed in claim 1, **characterized in that** the elastomer substrate is formed as a polyurethane substrate (9) and the textile intermediate layer (8) is applied to the outer side of the hollow polyurethane substrate and the silicone layer (7) is applied to said intermediate layer (8).

3. The method as claimed in claim 1 or 2, **characterized in that** a fleece layer, flock layer or velour layer is applied as the intermediate layer (8).

4. The method as claimed in one of claims 1 to 3, **characterized in that** the textile intermediate layer (8) is connected in an interlocking manner to the silicone layer (7) and the polyurethane substrate (9).

## Revendications

1. Procédé pour fabriquer une enveloppe d'un membre artificiel (1), dans lequel on applique une couche intermédiaire textile (8) sur la face extérieure d'un support creux en élastomère (9), **caractérisé en ce que** l'on applique une couche de silicone (7) sur la couche intermédiaire textile (8).

2. Procédé selon la revendication 1, **caractérisé en ce que** le support en élastomère (9) est réalisé sous forme de support en polyuréthane, et **en ce que** l'on applique sur la face extérieure du support creux en polyuréthane la couche intermédiaire textile (8), sur laquelle on applique la couche de silicone (7).

3. Procédé selon la revendication 1 ou 2, **caractérisé en ce que** l'on applique à titre de couche intermédiaire (8) une couche de peluche, une couche de flocage ou une couche en velours.

4. Procédé selon l'une des revendications 1 à 3, **caractérisé en ce que** la couche intermédiaire textile (8) est reliée en coopération de formes avec la couche de silicone (7) et avec le support en polyuréthane (9).
